# EUROPEAN PATENT SPECIFICATION

(11) **EP 2 726 076 B1**
(45) Date of publication and mention of the grant of the patent: **12.10.2016**
(21) Application number: 12732760.9
(22) Date of filing: 28.06.2012
(51) Int. Cl.: A61K 31/519, A61K 31/506, A61P 35/00

(54) **COMBINATION THERAPY**
KOMBINATIONSTHERAPIE
POLYTHÉRAPIE

(30) Priority: 01.07.2011 US 201161503641 P
(43) Date of publication of application: 07.05.2014
(73) Proprietor: Novartis AG, 4056 Basel (CH)
(72) Inventor: DOSHI, Shivang, Cambridge, Massachusetts 02139 (US); KIM, Sunkyu, Cambridge, Massachusetts 02139 (US); HAAS, Kristy, Cambridge, Massachusetts 02139 (US); KOVATS, Steven, Cambridge, Massachusetts 02139 (US)
(74) Representative: Lardans, Vinca Raymonde
(86) International application number: PCT/US2012/044607
(87) International publication number: WO 2013/006368

(56) References cited:
- WO-A2-2004/096224

## Description

### FIELD OF THE DISCLOSURE

A combination of a cyclin dependent kinase 4/6 (CDK4/6) inhibitor and an elective pan-fibroblast growth factor receptor (especially FGF-R3) kinase inhibitor for the treatment of solid tumors and hematological malignancies. This disclosure also relates to the use of the combination thereof, in the management of hyperproliferative diseases like cancer.

### RELATED BACKGROUND ART

Cyclin dependent kinase 4/6 (CDK4/6) inhibitors are described in, for example, WO2007/140222 and WO2010/020675.

Elective pan-fibroblast growth factor receptors (FGFR), especially FGF-R3 kinase inhibitors are described in, for example, WO2006/000420.

### BRIEF SUMMARY OF THE DISCLOSURE

The disclosure provides a combination comprising a first agent that inhibits the CDK4/6 pathway and a second agent that inhibits FGFR kinase(s). In another aspect, the disclosure provides combinations including pharmaceutical compositions comprising a therapeutically effective amount of a first agent that inhibits CDK4/6, a second agent that inhibits FGFR kinase(s), and a pharmaceutically acceptable carrier.

Furthermore, the present disclosure provides for the use of a therapeutically effective amount of a combination comprising a first agent that inhibits the CDK4/6 pathway and a second agent that inhibits FGFR kinase(s), or a pharmaceutically acceptable salt or pharmaceutical composition thereof, in the manufacture of a medicament for treating cancer.

The present disclosure has a therapeutic use in the treatment of various proliferative diseases.

The above combinations and compositions can be administered to a system comprising cells or tissues, as well as a human patient or and animal subject.

In one embodiment, the first agent that inhibits the CDK4/6 pathway is Compound A which is 7-Cyclopentyl-2-(5-piperazin-1-yl-pyridin-2-ylamino)-7H-pyrrolo[2,3-d]pyrimidine-6-carboxylic acid dimethylamide or pharmaceutically acceptable salt(s) thereof. Compound A is described by Formula A:

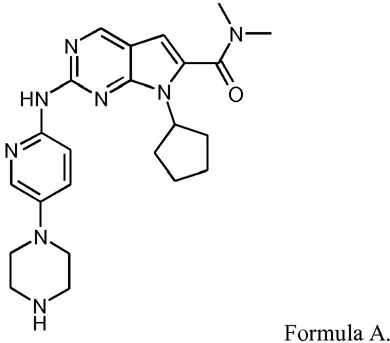

In another embodiment, the second agent that inhibits FGFR kinase is Compound B which is 3-(2,6-Dichloro-3,5-dimethoxy-phenyl)-1-{6-[4-(4-ethyl-piperazin-1-yl)-phenylamino]-pyrimidin-4-yl}-1-methyl-urea or pharmaceutically acceptable salt(s) thereof. Compound B is described by Formula B:

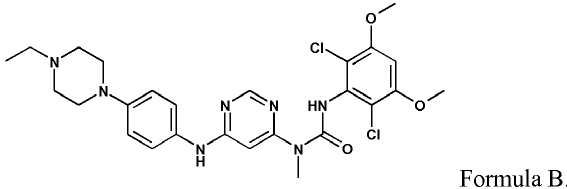

The preferred salt of Compound B is the mono-phosphate salt.

### BRIEF DESCRIPTION OF THE DRAWINGS

Figure 1 illustrates the results when the combination of Compound A and Compound B, is used to treat MultipleMyeloma KMS-11 cells. The resulting inhibition values were used by CHALICE software to generate Inhibition and ADD Excess Inhibition matrices, as well as the isobolograms.

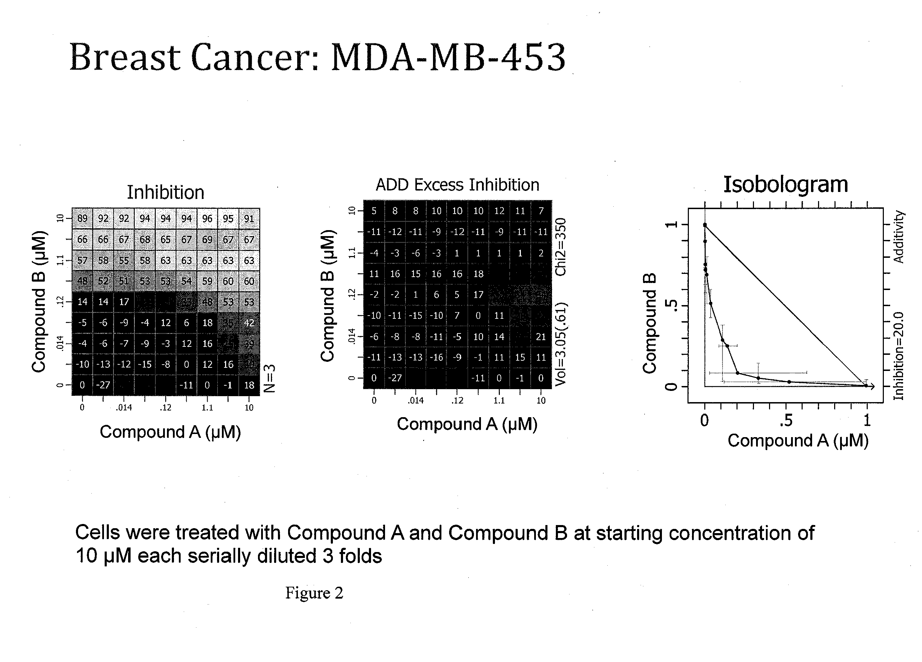

Figure 2 illustrates the results when the combination of Compound A and Compound B, is used to treat Breast Cancer: MDA-MB-453 cells. The resulting inhibition values were used by CHALICE software to generate Inhibition and ADD Excess Inhibition matrices, as well as the isobolograms.

### DETAILED DESCRIPTION OF THE DISCLOSURE

The disclosure provides a combination comprising a first agent that inhibits the CDK4/6 pathway and a second agent that inhibits FGFR kinase(s). In another aspect, the disclosure provides combinations including pharmaceutical compositions comprising a therapeutically effective amount of a first agent that inhibits CDK4/6, a second agent that inhibits FGFR kinase(s), and a pharmaceutically acceptable carrier.

Furthermore, the present disclosure provides for the use of a therapeutically effective amount of a combination comprising a first agent that inhibits the CDK4/6 pathway and a second agent that inhibits FGFR kinase(s), or a pharmaceutically acceptable salt or pharmaceutical composition thereof, in the manufacture of a medicament for treating cancer.

The present disclosure has a therapeutic use in the treatment of various proliferative diseases.

The above combinations and compositions can be administered to a system comprising cells or tissues, as well as a human patient or and animal subject.

In one embodiment, the first agent that inhibits the CDK4/6 pathway is Compound A which is 7-Cyclopentyl-2-(5-piperazin-1-yl-pyridin-2-ylamino)-7H-pyrrolo[2,3-d]pyrimidine-6-carboxylic acid dimethylamide or pharmaceutically acceptable salt(s) thereof. Compound A is described by Formula A:

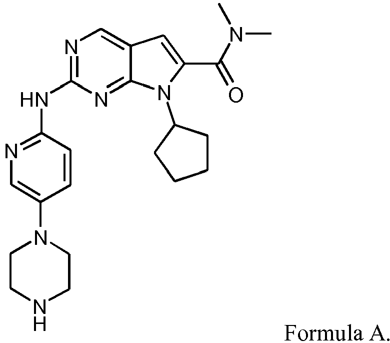

In another embodiment, the second agent that inhibits FGFR kinase is Compound B which is 3-(2,6-Dichloro-3,5-dimethoxy-phenyl)-1-{6-[4-(4-ethyl-piperazin-1-yl)-phenylamino]-pyrimidin-4-yl}-1-methyl-urea or pharmaceutically acceptable salt(s) thereof. Compound B is described by Formula B:

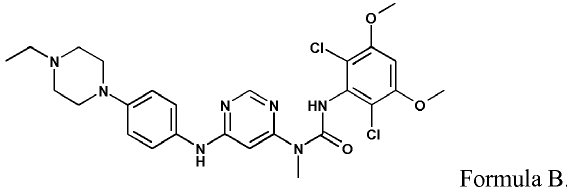

The preferred salt of Compound B is the mono-phosphate salt.

In another embodiment, the present disclosure includes a method of treating a hyperproliferative disease, preferably cancer. The compounds of the present disclosure inhibitors of CDK4/6 and therefore may be capable of treating diseases wherein the underlying pathology is (at least in part) mediated by CDK4/6. Such diseases include cancer and other diseases in which there is a disorder of cell proliferation, apoptosis, or differentiation.

Thus the combination of the present disclosure may be useful in the treatment of RB+ve (retinoblastoma protein positive) tumours, including tumours harbouring mutations in Ras, Raf, Growth Factor Receptors or over-expression of Growth Factor Receptors. The compounds of the present disclosure may also be useful in the treatment of tumours with amplifications of CDK4 and CDK6 genes as well as, tumours over-expressing cyclin partners of the cyclin dependent kinases. The compounds of the present disclosure may also be useful in the treatment of RB-ve tumours.

The combination of the present disclosure may also be useful in the treatment tumours with genetic aberrations that activate the CDK4/6 kinase activity. These include, but are not limited to, cancers with D-cyclin translocations such as mantle cell lymphoma and multiple myeloma, D-cyclin amplifications such as breast cancer and squamous cell esophageal cancer, CDK4 amplifications such as liposarcoma, CDK6 amplifications or overexpressions such as T-cell lymphoma and p16 inactivation such as melanoma, non-small cell lung cancer and pancreatic cancer.

The combination of the present disclosure may be useful in the treatment of cancers that have genetic aberrations in the upstream regulators of D-cyclins, where the defect results in an increase of D-cyclins abundance, can also be considered for treatment. These include, but are not limited to, acute myeloid leukemia with FLT3 activation, breast cancers with Her2/neu overexpression, ER dependency or triple negative phenotype, colon cancers with activating mutations of the MAPK, PI3K or WNT pathway, melanomas with activating mutations of MAPK pathway, non small cell lung cancers with activating aberrations of EGFR pathway and pancreatic cancers with activating aberrations of MAPK pathway including K-ras mutations.

Examples of cancers which may be treated with a compound of the present disclosure include but are not limited to, carcinoma, for example a carcinoma of the bladder, breast, colon (e.g. colorectal carcinomas such as colon adenocarcinoma and colon adenoma), kidney, epidermis, liver, lung(e.g. adenocarcinoma, small cell lung cancer and non-small cell lung carcinomas), oesophagus, gall bladder, ovary, pancreas (e.g. exocrine pancreatic carcinoma), stomach, cervix, thyroid, nose, head and neck, prostate, and skin (e.g. squamous cell carcinoma). Other examples of cancers that may be treated with a compound of the present disclosure include hematopoietic tumours of lymphoid lineage (e.g. leukemia, acute lymphocytic leukemia, mantle cell lymphoma, chronic lymphocytic leukaemia, B-cell lymphoma,(such as diffuse large B cell lymphoma), T-cell lymphoma, multiple myeloma, Hodgkin's lymphoma, non-Hodgkin's lymphoma, hairy cell lymphoma, and Burkett's lymphoma; hematopoietic tumours of myeloid lineage, for example acute and chronic myelogenous leukemias, myelodysplastic syndrome, and promyelocytic leukemia. Other cancers include thyroid follicular cancer; a tumour of mesenchymal origin, for example fibrosarcoma or habdomyosarcoma; a tumour of the central or peripheral nervous system, for example astrocytoma, neuroblastoma, glioma or schwannoma; melanoma; seminoma; teratocarcinoma; osteosarcoma; xeroderma pigmentosum; retinoblastoma; keratoctanthoma; thyroid follicular cancer; and Kaposi's sarcoma.

One group of cancers includes human breast cancers (e.g. primary breast tumours, node-negative breast cancer, invasive duct adenocarcinomas of the breast, non-endometrioid breast cancers); and endometrial cancers. Another sub-set of cancers wherein compounds having CDK4/6 inhibitory activity may be of particular therapeutic benefit comprises glioblastoma multiforme, T cell ALL, sarcomas, familial melanoma and melanoma.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

The phrase "therapeutically effective amount" is used herein to mean an amount sufficient to reduce by at least about 15 percent, preferably by at least 50 percent, more preferably by at least 90 percent, and most preferably prevent, a clinically significant deficit in the activity, function and response of the host. Alternatively, a therapeutically effective amount is sufficient to cause an improvement in a clinically significant condition/symptom in the host.

"Agent" refers to all materials that may be used to prepare pharmaceutical and diagnostic compositions, or that may be compounds, nucleic acids, polypeptides, fragments, isoforms, variants, or other materials that may be used independently for such purposes, all in accordance with the present disclosure.

The present disclosure includes all pharmaceutically acceptable isotopically-labeled compounds of the disclosure, i.e. compounds of Formula (I), wherein one or more atoms are replaced by atoms having the same atomic number, but an atomic mass or mass number different from the atomic mass or mass number usually found in nature.

Examples of isotopes suitable for inclusion in the compounds of the disclosure comprises isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of Formula (I), for example, those incorporating a radioactive isotope, are useful in drug and/or substrate tissue distribution studies. The radioactive isotopes tritium, *i.e.* ³H, and carbon-14, *i.e.* ¹⁴C, are particularly useful for this purpose in view of their ease of incorporation and ready means of detection.

Substitution with heavier isotopes such as deuterium, *i.e.* ² H, may afford certain therapeutic advantages resulting from greater metabolic stability, for example, increased *in vivo* half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of Formula (I) can generally be prepared by conventional techniques known to those skilled in the art or by processes analogous to those described in the accompanying Examples and Preparations using an appropriate isotopically-labeled reagents in place of the non-labeled reagent previously employed.

Compound A can be synthesized, for example, as described in WO2010/020675 or PCT/US2011/032062.

Compound B can be synthesized, for example, as described in WO2006/000420.

### EXAMPLES

### Example 1

Potential synergistic interactions between Compound A and Compound B combinations were assessed relative to the Loewe additivity model using CHALICE software, via a synergy score calculated from the differences between the observed and Loewe model values across the response matrix. Briefly, 9 titrating concentration ranging from 20 µM diluted serially three folds for Compound A and 2 µM diluted serially 3 folds for Compound B, including 0 µM, were used. In a 96 well plate, the 9 concentration points for each agent were mixed in a matrix format, generating 81 combinations. This plate was used to treat multiple myeloma KMS-11 cells, and the resulting inhibition values were used by CHALICE software to generate Inhibition and ADD Excess Inhibition matrices, as well as the isobolograms. A more detailed explanation of the technique and calculation can be found in Lehar et al. "Synergistic drug combinations improve therapeutic selectivity", Nat. Biotechnol. 2009, July; 27(7), 659-666, which is hereby incorporated by reference.

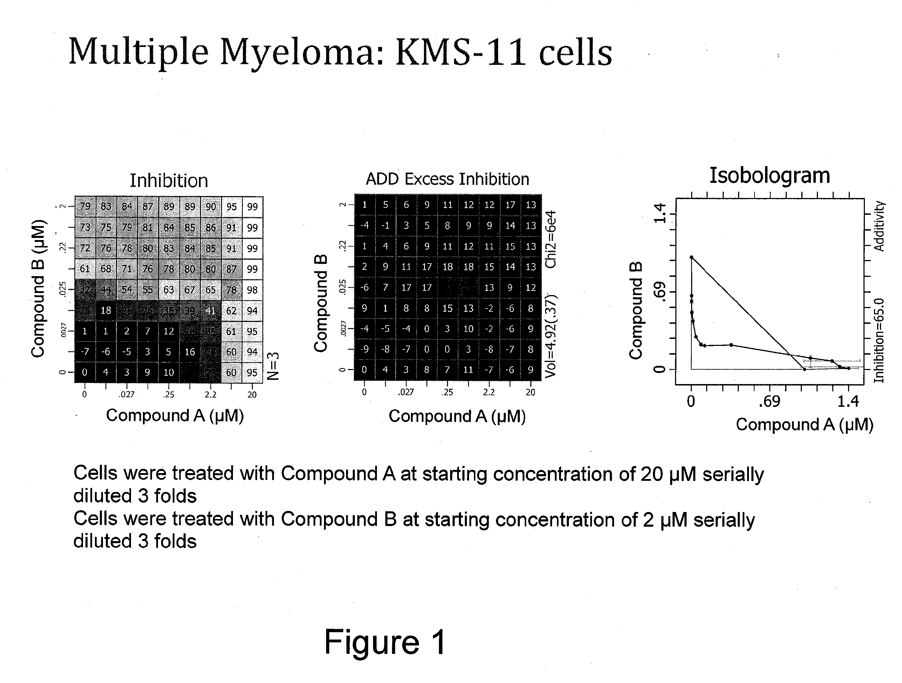

As illustrated by Figure 1, inhibition matrix shows the actual inhibition observed by the CTG assay at the respective concentrations of the compounds. ADD Excess inhibition shows the excess inhibition observed over the inhibition predicted by the Loewe additivity model. In addition to the matrices, one can use isobolograms to observe synergy. The inhibition level for each isobologram was chosen manually so as to observe the best synergistic effects. Isobologram was generated with Compound A concentrations shown on the y-axis and Compound B concentrations shown on the x-axis. A straight line connecting the Compound A and the Compound B concentrations which produce the chosen level of inhibition represented growth inhibitions that were strictly additive for the combinations. Plots placed below the line of additivity (more growth inhibition) represented synergistic growth inhibitions, while plots above the line of additivity (less growth inhibition) represented antagonistic growth inhibitions.

Synergic interaction is observed for the combination of Compound A and Compound B in the KMS-11 cells.

### Example 2

Potential synergistic interactions between Compound A and Compound B combinations were assessed relative to the Loewe additivity model using CHALICE software, via a synergy score calculated from the differences between the observed and Loewe model values across the response matrix. Briefly, 9 titrating concentration ranging from 10 µM diluted serially three folds for Compound A and 10 µM diluted serially 3 folds for Compound B, including 0 µM, were used. In a 96 well plate, the 9 concentration points for each agent were mixed in a matrix format, generating 81 combinations. This plate was used to treat breast cancer MDA-MB-453 cells, and the resulting inhibition values were used by CHALICE software to generate Inhibition and ADD Excess Inhibition matrices, as well as the isobolograms. A more detailed explanation of the technique and calculation can be found in Lehar et al. "Synergistic drug combinations improve therapeutic selectivity", Nat. Biotechnol. 2009, July; 27(7), 659-666, which is hereby incorporated by reference.

As illustrated by Figure 1, inhibition matrix shows the actual inhibition observed by the CTG assay at the respective concentrations of the compounds. ADD Excess inhibition shows the excess inhibition observed over the inhibition predicted by the Loewe additivity model. In addition to the matrices, one can use isobolograms to observe synergy. The inhibition level for each isobologram was chosen manually so as to observe the best synergistic effects. Isobologram was generated with Compound A concentrations shown on the y-axis and Compound B concentrations shown on the x-axis. A straight line connecting the Compound A and the Compound B concentrations which produce the chosen level of inhibition represented growth inhibitions that were strictly additive for the combinations. Plots placed below the line of additivity (more growth inhibition) represented synergistic growth inhibitions, while plots above the line of additivity (less growth inhibition) represented antagonistic growth inhibitions.

Synergic interaction is observed for the combination of Compound A and Compound B in the MDA-MB-453 cells.

## Claims

1. A combination comprising a first agent that is a cyclin dependent kinase 4 or cyclin dependent kinase 6 (CDK4/6) inhibitor and a second agent that is an FGFR kinase inhibitor.

2. The combination of claim 1 wherein the first agent is Compound A described by Formula A: 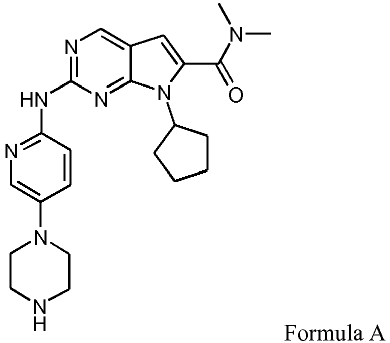 or a pharmaceutically acceptable salt thereof.

3. The combination of claim 1 or 2, wherein the second agent is Compound B described by Formula B: 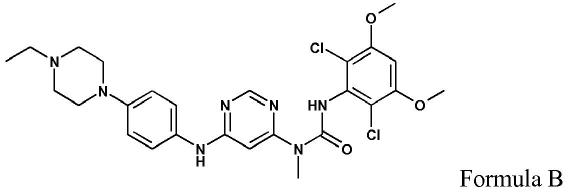 or a pharmaceutically acceptable salt thereof.

4. The use of the combination according to any one of claims 1-3 for the manufacture of a medicament, wherein the medicament is for treating cancer.

5. The use of claim 4, wherein the cancer is a solid tumor cancer.

6. The use of claim 5, wherein the cancer is pancreatic cancer, breast cancer, mantle cell lymphoma, non small cell lung cancer, melanoma, esophageal cancer, liposarcoma, multiple myeloma, T-cell leukemia, renal cell carcinoma, glioblastoma, hepatocellular carcinoma, gastric cancer, lung cancer or colon cancer.

7. The use of claim 6, wherein the cancer is pancreatic cancer, breast cancer, or mantle cell lymphoma.

8. The use of claim 6, wherein the cancer is mantle cell lymphoma.

9. The combination according to any one of claims 1-3 for use in the treatment of cancer.

10. The combination according to claim 9 wherein the cancer is a solid tumor cancer.

11. The combination according to claim 10, wherein the cancer is pancreatic cancer, breast cancer, mantle cell lymphoma, non small cell lung cancer, melanoma, esophageal cancer, liposarcoma, multiple myeloma, T-cell leukemia, renal cell carcinoma, glioblastoma, hepatocellular carcinoma, gastric cancer, lung cancer or colon cancer.

12. The combination according to claim 11, wherein the cancer is pancreatic cancer, breast cancer, or mantle cell lymphoma.

13. The combination according to claim 11, wherein the cancer is mantle cell lymphoma.

## Patentansprüche

1. Kombination, umfassend ein erstes Mittel, bei dem es sich um einen Inhibitor der cyclinabhängigen Kinase 4 (Cyclin Dependent Kinase 4) oder der cyclinabhängigen Kinase 6 (Cyclin Dependent Kinase 6) (CDK4/6-Inhibitor) handelt, und ein zweites Mittel, bei dem es sich um einen FGFR-Kinase-Inhibitor handelt.

2. Kombination nach Anspruch 1, wobei es sich bei dem ersten Mittel um Verbindung A, beschrieben durch Formel A: 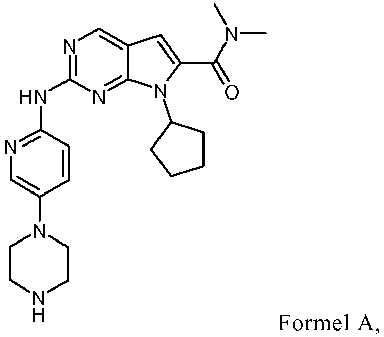 oder ein pharmazeutisch unbedenkliches Salz davon handelt.

3. Kombination nach Anspruch 1 oder 2, wobei es sich bei dem zweiten Mittel um Verbindung B, beschrieben durch Formel B: 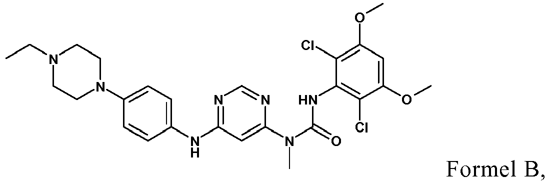 oder ein pharmazeutisch unbedenkliches Salz davon handelt.

4. Verwendung der Kombination nach einem der Ansprüche 1-3 zur Herstellung eines Medikaments, wobei das Medikament für die Behandlung von Krebs bestimmt ist.

5. Verwendung nach Anspruch 4, wobei es sich bei Krebserkrankung um einen festen Krebstumor handelt.

6. Verwendung nach Anspruch 5, wobei es sich bei der Krebserkrankung um Bauchspeicheldrüsenkrebs, Brustkrebs, Mantelzelllymphom, nichtkleinzelligen Lungenkrebs, Melanom, Speiseröhrenkrebs, Liposarkom, multiples Myelom, T-Zell-Leukämie, Nierenzellkarzinom, Glioblastom, hepatozelluläres Karzinom, Magenkrebs, Lungenkrebs oder Dickdarmkrebs handelt.

7. Verwendung nach Anspruch 6, wobei es sich bei der Krebserkrankung um Bauchspeicheldrüsenkrebs, Brustkrebs oder Mantelzelllymphom handelt.

8. Verwendung nach Anspruch 6, wobei es sich bei der Krebserkrankung um Mantelzelllymphom handelt.

9. Kombination nach einem der Ansprüche 1-3 zur Verwendung bei der Behandlung von Krebs.

10. Kombination nach Anspruch 9, wobei es sich bei der Krebserkrankung um einen festen Krebstumor handelt.

11. Kombination nach Anspruch 10, wobei es sich bei der Krebserkrankung um Bauchspeicheldrüsenkrebs, Brustkrebs, Mantelzelllymphom, nichtkleinzelligen Lungenkrebs, Melanom, Speiseröhrenkrebs, Liposarkom, multiples Myelom, T-Zell-Leukämie, Nierenzellkarzinom, Glioblastom, hepatozelluläres Karzinom, Magenkrebs, Lungenkrebs oder Dickdarmkrebs handelt.

12. Kombination nach Anspruch 11, wobei es sich bei der Krebserkrankung um Bauchspeicheldrüsenkrebs, Brustkrebs oder Mantelzelllymphom handelt.

13. Kombination nach Anspruch 11, wobei es sich bei der Krebserkrankung um Mantelzelllymphom handelt.

## Revendications

1. Combinaison comprenant un premier agent qui est un inhibiteur de kinase cycline-dépendante 4 ou de kinase cycline-dépendante 6 (CDK4/6) et un deuxième agent qui est un inhibiteur de FGFR kinase.

2. Combinaison de la revendication 1 dans laquelle le premier agent est le composé A décrit par la formule A : 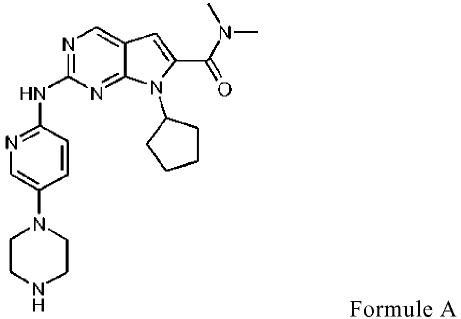 ou un sel pharmaceutiquement acceptable de celui-ci.

3. Combinaison de la revendication 1 ou 2, dans laquelle le deuxième agent est le composé B décrit par la formule B : 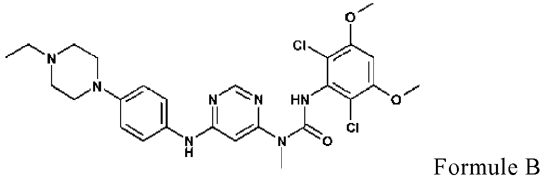 ou un sel pharmaceutiquement acceptable de celui-ci.

4. Utilisation de la combinaison selon l'une quelconque des revendications 1 à 3 pour la fabrication d'un médicament, le médicament étant pour traiter un cancer.

5. Utilisation de la revendication 4, dans laquelle le cancer est un cancer à tumeur solide.

6. Utilisation de la revendication 5, dans laquelle le cancer est un cancer pancréatique, un cancer du sein, un lymphome à cellules du manteau, un cancer du poumon non à petites cellules, un mélanome, un cancer de l'oesophage, un liposarcome, un myélome multiple, une leucémie à cellules T, un carcinome à cellules rénales, un glioblastome, un carcinome hépatocellulaire, un cancer gastrique, un cancer du poumon ou un cancer du côlon.

7. Utilisation de la revendication 6, dans laquelle le cancer est un cancer pancréatique, un cancer du sein, ou un lymphome à cellules du manteau.

8. Utilisation de la revendication 6, dans laquelle le cancer est un lymphome à cellules du manteau.

9. Combinaison selon l'une quelconque des revendications 1 à 3 pour utilisation dans le traitement d'un cancer.

10. Combinaison selon la revendication 9 dans laquelle le cancer est un cancer à tumeur solide.

11. Combinaison selon la revendication 10, dans laquelle le cancer est un cancer pancréatique, un cancer du sein, un lymphome à cellules du manteau, un cancer du poumon non à petites cellules, un mélanome, un cancer de l'oesophage, un liposarcome, un myélome multiple, une leucémie à cellules T, un carcinome à cellules rénales, un glioblastome, un carcinome hépatocellulaire, un cancer gastrique, un cancer du poumon ou un cancer du côlon.

12. Combinaison selon la revendication 11, le cancer étant un cancer pancréatique, un cancer du sein, ou un lymphome à cellules du manteau.

13. Combinaison selon la revendication 11, le cancer étant un lymphome à cellules du manteau.
